(19) Europäisches Patentamt
European Patent Office
Office européen des brevets

(11) Publication number: **0 152 470 B1**

(12) # EUROPEAN PATENT SPECIFICATION

(45) Date of publication of patent specification: **12.10.88**

(51) Int. Cl.[4]: **C 07 C 2/00**, C 07 C 2/76, C 07 C 15/02

(21) Application number: **84903233.9**

(22) Date of filing: **10.08.84**

(88) International application number: **PCT/US84/01284**

(87) International publication number: **WO 85/00804 28.02.85 Gazette 85/05**

(54) **METHANE CONVERSION.**

(30) Priority: **12.08.83 US 522937**
**12.08.83 US 522936**
**16.04.84 US 600654**

(43) Date of publication of application: **28.08.85 Bulletin 85/35**

(45) Publication of the grant of the patent: **12.10.88 Bulletin 88/41**

(84) Designated Contracting States: **BE DE FR NL**

(56) References cited:
**GB-A- 255 829**
**US-A- 258 608**
**US-A-4 205 194**
**US-A-4 239 658**
**US-A-4 444 984**
**US-A-4 450 310**

**Journce of the Chinese Chemical Society, Published 1981, Treliant Fang and Chium Ti Yeh, Catalytic Pyrolysis of Methane, See pages 265-273.**

(73) Proprietor: **ATLANTIC RICHFIELD COMPANY**
**515 South Flower Street**
**Los Angeles California 90071 (US)**

(72) Inventor: **JONES, C., Andrew**
**24 Clearbrook Road**
**Newtown Square, PA 19073 (US)**
Inventor: **SOFRANKO, John, A.**
**Box 197, Line Road RD4**
**Malvern, PA 19355 (US)**

(74) Representative: **Cropp, John Anthony David et al**
**MATHYS & SQUIRE 10 Fleet Street**
**London, EC4Y 1AY (GB)**

(56) References cited:
**Journal of Catalysis, Published 1982, G.E. Keller and M.M. Bhasin, Synthesis of Ethylene via Oxidative Coupling of Methane, See pages 9-18.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 152 470 B1

**Description**

Technical field

This invention relates to synthesis of hydrocarbons from a methane source. A particular application of this invention is a method for converting natural gas to a more readily transportable material.

Background art

A major source of methane is natural gas. Other sources of methane have been considered for fuel supply, e.g., the methane present in coal deposits or formed during mining operations. Relatively small amounts of methane are also produced in various petroleum processes.

The composition of natural gas at the well-head varies but the major hydrocarbon present is methane. For example, the methane content of natural gas may vary within the range from about 4 to about 95 volume percent. Other constituents of natural gas include ethane, propane, butanes, pentane (and heavier hydrocarbons), hydrogen sulfide, carbon dioxide, helium and nitrogen.

Natural gas is classified as dry or wet depending upon the amount of condensable hydrocarbons contained in it. Condensable hydrocarbons generally comprise $C_3$+ hydrocarbons although some ethane may be included. Gas conditioning is required to alter the composition of wellhead gas, processing facilities usually being located in or near the production fields. Conventional processing of wellhead natural gas yields processed natural gas containing at least a major amount of methane.

Large-scale use of natural gas often requires a sophisticated and extensive pipeline system. Liquefaction has also been employed as a transporation means, but processes for liquefying transporting, and revaporizing natural gas are complex, energy-intensive and require extensive safety precautions. Transport of natural gas has been a continuing problem in the exploitation of natural gas resources. It would be extremely valuable to be able to convert methane (e.g., natural gas) to more readily handleable or transportable products. Moreover, direct conversion to olefins such as ethylene or propylene would be extremely valuable to the chemical industry.

Recently, it has been discovered that methane may be converted to higher hydrocarbons by a process which comprises contacting methane and an oxidative synthesizing agent at synthesizing conditions (e.g., at a temperature selected within the of from about 500° to about 1000°C). Oxidative synthesizing agents are compositions having as a principal component at least one oxide of at least one metal which compositions produce $C_2$+ hydrocarbon products, co-product water, and a composition comprising a reduced metal oxide when contacted with methane at synthesizing conditions. Reducible oxides of several metals have been identified which are capable of converting methane to higher hydrocarbons. In particular, oxides of manganese, tin, indium, germanium, lead, antimony and bismuth are most useful. See commonly-assigned U.S. Patent Nos. 4,443,649; 4,444,984; 4,443,648; 4,443,645; 4,443,647; 4,443,644; and 4,443,646, the entire contents of which are incorporated herein by reference.

U.S. Patent No. 4,205,194 proposes converting methane to higher hydrocarbons by contacting the methane with a catalyst reagent comprising (1) a Group VIII noble metal having an atomic number of 45 or greater, nickel, or a Group I-B noble metal having an atomic number of 47 or greater; (2) a Group VI-B metal oxide which is capable of being reduced to a lower oxide, or admixture of metal oxides which includes one or more of such metal oxides; and, (3) a Group II-A metal or alkaline earth metal, composited with a suitably passivated, spinel-coated refractory support, notably an inorganic oxide support, preferably alumina.

As noted, the reaction products of such processes are mainly ethylene, ethane, other light hydrocarbons, carbon oxides, coke and water. It would be beneficial to these oxidative synthesis processes to reduce selectivities to carbon oxides and coke.

Accordingly, an object of this invention is to provide an improved process for converting methane to higher hydrocarbons. A further object of this invention is an improved oxidative synthesizing agent, one capable of converting methane with reduced by-product selectivities. A still further object of this invention is an oxidative synthesising agent with improved stability, an agent that maintains desirable conversion properties for longer periods of time.

Other aspects, objects and the several advantages of this invention will become apparent to those skilled in the art upon reading this disclosure and the appended claims.

According to the present invention, there is provided a method for converting methane to higher hydrocarbon products which comprises contacting a gas comprising methane at synthesising conditions with at least one reducible oxide of at least one metal, which oxide when contacted with methane at synthesising conditions is reduced and produces higher hydrocarbon products and water, characterised in that the contacting is conducted in the presence of a promoting amount of at least one promoter (a) selected from alkali metals and compounds thereof optionally together with at least one promoter (b) selected from alkaline earth metals and compounds thereof, said reducible oxide and said promoter or promoters optionally being associated with a support material.

Also according to the invention, there is provided a method for converting methane to higher hydrocarbon products which comprises contacting a gas comprising methane at synthesising conditions with at least one reducible oxide of at least one metal, which oxide when contacted with methane at synthesising conditions is reduced and produces higher hydrocarbon products and water, characterised in

that the contacting is conducted in the presence of a promoting amount of at least one promoter selected from alkaline earth metals and compounds thereof, said contacting being effected in the absence of catalytically effective Ni, Rh, Pd, Ag, Os, Ir, Pt, Au and compounds thereof, and said reducible oxide and said promoter optionally being associated with a support material.

Alkali metals are selected from the group consisting of Li, Na, K, Rb and Cs. Preferred alkali metal promoters are Na, K and Li. Alkaline earth metals are selected from the group consisting of Mg, Ca, Sr and Ba. Preferred alkaline earth metal promoters are Mg and Ca. Magnesium is a particularly perferred alkaline earth metal promoter. Sodium is a particularly preferred promoter. Preferred reducible metal oxides are reducible oxides of Mn, Sn, In, Ge, Pb, Sb and Bi. Particularly preferred reducible metal oxides are reducible oxides of manganese.

Where the promoter is selected from alkali metals and compounds thereof, the reducible oxide and the promoter may be associated with a support comprising at least one member of the group consisting of alkaline earth metals and compounds thereof.

Alkaline earth metals are selected from the group consisting of Mg, Ca, Sr and Ba. Magnesia is a particularly preferred support.

In a separate, distinct embodiment of the present invention, it has been found that the stability of the promoted oxidative synthesizing agent is enhanced by incorporating a stabilizing amount of phosphorus into the composition.

The present process is distinguished from previously known pyrolytic methane conversion processes by the use of the aforementioned reducible oxides to synthesize higher hydrocarbons from methane with coproduction of water, rather than hydrogen.

The present process is distinguished from previously suggested methane conversion processes which rely primarily on interactions between methane and at least one of nickel and the noble metals, such as rhodium, palladium, silver, osmium, iridium, platinum and gold. An example of this type of process is disclosed in U.S. Patent 4,205,194. The present process does not require that methane be contacted with one or more of nickel and such noble metals and compounds thereof.

Moreover, in a preferred embodiment, such contacting is carried out in the absence of catalytically effective nickel and the noble metals and compounds thereof to minimize the deleterious catalytic effects of such metals and compounds thereof. For example, at the conditions, e.g., temperatures, useful for the contacting step of the present invention, these metals when contacted with methane tend to promote coke formation, and the metal oxides when contacted with methane tend to promote formation of combustion products ($CO_x$) rather than the desired hydrocarbons. The term "catalytically effective" is used herein to identify that quantity of one or more of nickel and the noble metals and compounds thereof which when present substantially changes the distribution of products obtained in the contacting step of this invention relative to such contacting in the absence of such metals and compounds thereof.

## Brief description of the drawings

Figure 1 is a plot of $C_2$+ hydrocarbon product selectivity vs. methane conversion for the tests described in Example 19.

Figure 2 is a plot of methane conversion vs. run time (or cycles) for the extended life tests described in Example 20.

## Best modes for carrying out the invention

Oxidative synthesizing agents comprise at least one oxide of at least one metal, which oxides when contacted with methane at synthesizing conditions (e.g., at a temperature selected within the range of 500 to 1000°C) produce higher hydrocarbon products, co-product water, and a reduced metal oxide. The composition thus contains at least one reducible oxide of at least one metal. The term "reducible" is used to identify those oxides of metals which are reduced by contacting methane at synthesizing conditions (e.g., at temperatures selected within the range of 500—1000°C). The term "oxide(s) of metal(s)" includes: (1) one or more metal oxides (i.e., compounds described by the general formula $M_xO_y$ wherein M is a metal and the subscripts x and y designate the relative atomic proportions of metal and oxygen in the composition) and/or (2) one or more oxygen-containing metal compounds, provided that such oxides and compounds have the capability of performing to produce higher hydrocarbon products as set forth herein.

Preferred oxidative synthesizing agents comprise reducible oxides of metal selected from the group consisting of Mn, Sn, In, Ge, Sb, Pb, and Bi and mixtures thereof. Particularly preferred oxidative synthesizing agents comprise a reducible oxide of manganese and mixtures of a reducible oxide of manganese with other oxidative synthesizing agents.

According to the invention, the oxidative synthesising agent is used with a promoter. Where the promoter comprises at least one alkali metal, the atomic ratio in which these materials are combined to form the synthesising agent is not narrowly critical. However, the preferred atomic ratio of the reducible oxide component (expressed as the metal, e.g. Mn) to the alkali metal component (expressed as the metal, e.g. Na) is within the range of 0.1—100:1, more preferably within the range of 0.3—10:1.

Where at least one alkaline earth metal or compound thereof is employed as a promoter, the atomic ratio in which the materials are combined to form the synthesizing agent is not narrowly critical. However, the preferred atomic ratio of the reducible oxide component (expressed as the metal, e.g., Mn) to the

alkaline earth component (expressed as the metal, e.g. Mg) is within the range of 0.01—100:1 more preferably within the range of 0.1—10:1.

The promoted oxidative synthesizing agent may also contain at least one phosphorus component in a distinct, preferred embodiment. The amount of the phosphorus component contained in the synthesizing agent is again not narrowly critical. The atomic ratio of phosphorus to the reducible oxide component (e.g., Mn) is preferably less than about 10:1. More preferably this ratio is within the range of 0.1—0.5:1.

One preferred oxidative synthesizing agent used in the process of this invention may be further expressed by the following empirical formula:

$$A_a\ B_b\ P_c\ O_d$$

wherein A is selected from the group consisting of Mn, Sn, In, Ge, Pb, Sb, Bi and mixtures thereof; B is selected from the group consisting of alkali metals and mixtures thereof; a to d indicate the atomic ratio of each component; and when a is 10, b is within the range of 1—33, c is within the range of 0—20, and d has a value which is determined by the valence and proportions of the other elements present. These components may be associated with a support material as described below.

Another preferred oxidative synthesizing agent used in the process of this invention may be further expressed by the following empirical formula:

$$A_a\ B_b\ P_c\ O_d$$

wherein A is selected from the group consisting of Mn, Sn, In, Ge, Pb, Sb, Bi and mixtures thereof; B is selected from the group consisting of alkaline earth metals and mixtures thereof; a to d indicate the atomic ratio of each component; and when a is 10, b is within the range of 1—100, c is within the range of 0—100, and d has a value which is determined by the valence and proportions of the other elements present. These components may be associated with a support material as described below.

The promoted oxidative synthesizing agent may be supported by or diluted with conventional support materials such as silica, alumina, titania, zirconia and combinations thereof. Moreover, the support material may comprise the promoter itself (e.g., suitable supports include $Na_2O$, $K_2O$, MgO, CaO, BaO).

In one preferred, distinct embodiment of this invention, an alkali metal promoted oxidative synthesizing agent is associated with a support comprising at least one member of the group consisting of alkaline earth metals and compounds thereof. Preferred support materials comprise MgO, CaO, BaO and mixtures thereof. MgO is a particularly preferred support.

The promoted oxidative synthesizing agent can be prepared by any suitable method. Conventional methods such as precipitation, co-precipitation, impregnation, or dry-mixing can be used. Supported solids may be prepared by methods such as adsorption, impregnation, precipitation, co-precipitation, and dry-mixing. Thus, a compound of Mn, Sn, In, Ge, Pb, Sb and/or Bi; a compound of an alkali and/or alkaline earth metal; and optionally a compound of phosphorus can be combined in any suitable way. When phosphorus is incorporated in the agent, it is desirable to provide it in the form of a phosphate of alkali and/or alkaline earth metal. Substantially any compound of these elements can be employed in the preparation of the promoted synthesizing agent.

A suitable method of preparation is to impregnate a support with solutions of compounds of the desired metals. Compounds useful for impregnation include the acetates, acetlyacetonates, oxides, carbides, carbonates, hydroxides, formates, oxalates, nitrates, phosphates, sulfates, sulfides, tartrates, fluorides, chlorides, bromides, or iodides. After impregnation the preparation is dried in an oven to remove solvent and the dried solid is prepared for use by calcining, preferably in air at a temperature selected within the range of 300 to 1200°C. Particular calcination temperatures will vary depending upon the particular metal compound or compounds employed.

If phosphorus is used, the alkali and/or alkaline earth metal and phosphorus are preferably added to the composition as compounds containing both P and alkali and/or alkaline earth metals. Examples are the orthophosphates, metaphosphates, and pyrophosphates of alkali and/or alkaline earth metals. Pyrophosphates have been found to give desirable results. The alkali and/or alkaline earth metal and the phosphorus can be incorporated into the promoted synthesizing agent as separate compounds. Suitable phosphorus compounds useful for preparing the compositions include orthophosphoric acid, ammonium phosphates and ammonium hydrogenphosphates.

Regardless of how the components of the synthesizing agent are combined, the resulting composite generally will be dried and calcined at elevated temperatures in an oxygen-containing gas (e.g., air) prior to use in the process of this invention.

In addition to methane, the feedstock employed in the method of this invention may contain other hydrocarbon or non-hydrocarbon components, although the methane content should typically be within the range of 40 to 100 volume percent, preferably from 80 to 100 volume percent, more preferably from 90 to 100 volume percent.

Operating temperatures for the contacting of methane-containing gas and the promoted oxidative synthesizing agent are selected within the range of 500 to 1000°C, the particular temperature selected depending upon the particular reducible metal oxide(s) employed in the promoted oxidative synthesizing

agent. For example, reducible oxides of certain metals, may require operating temperatures below the upper part of the recited range to minimize sublimation of volatilization of the metals (or compounds thereof) during methane contact. Examples are: (1) reducible oxides of indium, (operating temperatures will preferably not exceed about 850°C); (2) reducible oxides of germanium (operating temperatures will preferably not exceed about 850°C); and (3) reducible oxides of bismuth (operating temperatures will preferably not exceed about 850°C).

Operating pressures for the methane contacting step are not critical to the presently claimed invention. However, both general system pressure and partial pressure or metahne have been found to effect overall results. Preferred operating pressures are 1 within the range of 1 to 30 atmospheres.

Contacting methane and a promoted oxidative synthesizing agent to form higher hydrocarbons from methane also produces a reduced metal oxide and co-product water. The exact nature of the reduced metal oxides are unknown, and so are referred to herein as "reduced metal oxides". Regeneration of a reducible metal oxide is readily accomplished by contacting reduced compositions with oxygen (e.g., an oxygen-containing gas such as air) at a temperature selected within the range of 300 to 1200°C, the particular temperatures selected depending on the metal(s) included in the oxidative synthesizing agent.

In carrying out the present process, a single reactor apparatus containing a fixed bed of solids may be used with intermittent or pulsed flow of a first gas comprising methane and a second gas comprising oxygen (e.g., oxygen, oxygen diluted with an inert gas, or air, preferably air). The methane contacting step and the oxygen contacting step are more preferably performed in physically separate zones with solids recirculating between the two zones.

Thus, a suitable method for synthesizing hydrocarbons from a methane source comprises: (a) contacting a gas comprising methane and particles comprising a promoted oxidative synthesizing agent to form higher hydrocarbon products, co-product water, and reduced metal oxide; (b) removing particles comprising reduced synthesizing agents from the first zone and contacting the reduced particles in a second zone with an oxygen-containing gas to form particles comprising a promoted oxidative synthesizing agent; and (c) returning the particles produced in the second zone to the first zone. The steps are preferably repeated at least periodically, and more preferably the steps are continuous. In the more preferred embodiment solids are continuously circulated between at least one methane-contact zone and at least one oxygen-contact zone.

Particles comprising a promoted oxidative synthesizing agent which are contacted with methane may be maintained as fluidized, ebullating, or entrained beds of solids. Preferably methane is contacted with a fluidized bed of solids.

Similarly, particles comprising reduced metal oxide which are contacted with oxygen may be maintained as fluidized, ebullating or entrained beds of solids. Preferably oxygen is contacted with a fluidized bed of solids.

In the more preferred embodiment of the present invention, methane feedstock and particles comprising a promoted oxidative synthesizing agent are continuously introduced into a methane contact zone maintained at synthesizing conditions. Synthesizing conditions include the temperatures and pressures described above. Gaseous reaction products from the methane contact zone (separated from entrained solids) are further processed—e.g., they are passed through a fractionating system wherein the desired hydrocarbon products are separated from unconverted methane and combustion products. Unconverted methane may be recovered and recycled to the methane contact zone.

Particles comprising reduced metal oxide are contacted with oxygen in an oxygen contact zone for a time sufficient to oxidize at least a portion of the reduced oxide to produce a reducible metal oxide and to remove, i.e., combust, at least a portion of any carbonaceous deposit which may form on the particles in the methane contact zone. The conditions of the oxygen contact zone will preferably include a temperature selected within the range of 300 to 1200°C, pressures of up to about 30 atmospheres, and average particle contact time within the range of 1 to 120 minutes. Sufficient oxygen is preferably provided to oxidize all reduced metal oxide to produce a reducible oxide and to completely combust any carbonaceous deposit material deposited on the particles. At least a portion of the particles comprising a promoted oxidative synthesizing agent which are produced in the oxygen contact zone are returned to the methane contact zone.

The rate of solids withdrawal from the methane contact zone is desirably balanced with the rate of solids passing from the oxygen contact zone to the methane contact zone so as to maintain a substantially constant inventory of particles in the methane contact zone, thereby enabling steady state operation of synthesizing system.

The invention is further illustrated by reference to the following examples.

Methane-contact runs were made at about atmospheric pressure in quartz tube reactors (12 mm. inside diameter) packed with 7 ml. of catalyst. The reactors were brought up to temperature under a flow of nitrogen which was switched to methane at the start of the run. Unless otherwise indicated, all methane-contact runs described in the following examples had a duration of 2 minutes. At the end of each methane-contact run, the reactor was flushed with nitrogen and the solids were regenerated under a flow of air (usually at 800°C for 30 minutes). The reactor was then again flushed with nitrogen and the cycle repeated. The results reported below are based on the cumulative sample collected after the contact solid has "equilibrated"—i.e., after the aberrant characteristics of the fresh solid have dissipated. This has been

done to allow more meaningful comparisons to be made between the various contact agents within the scope of this invention and further comparaisons between contact agents within the scope of this invention and other contact agents. Three to six cycles of methane-contact and regeneration are generally sufficient to equilibrate the solids.

Experimental results reported below include conversions and selectivities calculated on a carbon mole basis.

Space velocities are reported as gas hourly space velocities (hr.$^{-1}$) and are identified as "GHSV" in the Examples.

Examples 1—3 and Comparative Example A

Supported manganese oxides promoted by various alkali metals were made by impregnating a silica support with the appropriate amount of manganese (as manganese acetate) and the appropriate amount of alkali or alkaline earth metal (also as the acetate) from water solutions. The support was Houdry HSC 534 silica. The impregnated solids were dried at 110°C for 4 hours and then calcined in air at 700°C for 16 hours. All calcined solids contained 10 wt.% Mn and each contained the same mole % of alkali metal. Results reported below in Table I are based on analyses of cumulative samples collected during the third, two-minute, methane-contact run for each promoted oxidative synthesizing agent. Run conditions were 800°C and 860 GHSV. Table I also shows results obtained over an unpromoted oxidative synthesizing agent.

TABLE I

| Example | Oxidative Sythesizing agent | %$CH_4$ conversion | %$C_{2-7}$ selectivity |
|---|---|---|---|
| 1 | 1.7%Na/10%Mn/$SiO_2$ | 15.2 | 82 |
| 2 | 2.9%K/10%Mn/$SiO_2$ | 14.0 | 58 |
| 3 | 0.5%Li/10%Mn/$SiO_2$ | 10.9 | 74 |
| A | 10%Mn/$SiO_2$ | 7.6 | 56 |

Examples 4—5 and Comparative Example B

Several unsupported bulk oxides were prepared to exemplify the enhanced selectivity obtained when promoted oxidative synthesizing agents are used in the absence of a support material. The first—described as "NaMn-oxide"— was prepared by calcining sodium permanganate ($NaMnO_4$) in air at 800°C for several hours. X-ray diffraction analysis of the calcined solid indicated the presence of $NaMnO_2$ and $Na_{0.7}MnO_2$. The second bulk oxide—described as "LiMn-oxide"—was similarly prepared from $Li_2MnO_3$. For comparison, bulk manganese oxide was similarly prepared from manganese acetate $Mn(COOCH_3)_2 \cdot 4H_2O$). X-ray diffraction analysis of this calcined solid indicated the presence of $Mn_2O_3$. Results reported below in Table II are based on analyses of cumulative samples collected during the third, two-minute, methane-contact run for each of these bulk oxide contact agents. Run conditions were 800°C and 860 GHSV.

TABLE II

| Example | Unsupported oxidative synthesizing agent | % $CH_4$ conversion | % $C_{2-7}$ selectivity |
|---|---|---|---|
| 4 | NaMn oxide | 19.2 | 78.6 |
| 5 | LiMn oxide | 25.7 | 44.6 |
| B | Mn oxide | 38.0 | 1.2 |

Examples 6—8

A sodium silicate solution (31.7 grams) containing 9 wt. % $Na_2O$, 29 wt. % $SiO_2$ and 62 wt. % $H_2O$ was diluted with 100 ml. $H_2O$. Manganese acetate (37.4 grams of $Mn(COOCH_3)_2 \cdot 4H_2O$) was dissolved in 300 ml. $H_2O$. The first solution was slowly added to the second solution with stirring to form a precipitate. Stirring continued for 1½ hours. This preparation was repeated twice to form three batches of the mixture.

A first portion of the mixture was slowly dried without boiling. It was then further dried at 110°C for 12 hours, and then calcined by heating to 800°C at a rate of 1°C/minute in air and holding at 800°C for 12 hours. Analysis by atomic absorption showed 9% Na in the final product. Methane conversion results obtained with this solid are presented as Example 6 in Table III below.

A second portion of the mixture was allowed to settle for 12 hours and was then decanted. The settled, decanted precipitate was washed twice with $H_2O$ and then dried slowly without boiling. It was further dried at 110°C for 12 hours and then calcined as described above. Analysis by atomic absorption showed 2% Na in the final product. Methane conversion results obtained with this solid are presented as Example 7 in Table III below.

A third portion of the mixture, was allowed to settle for 12 hours and was then decanted. The settled, decanted precipitate was washed twice with $H_2O$. An additional 37.4 grams of magnanese acetate was dissolved in 300 ml. $H_2O$ and this solution was added to the solid, the mixture stirred for 1/2 hour and then dried without boiling. It was further dried at 110°C for 12 hours and then calcined as described above. Analysis by atomic absorption showed 2% Na in the final product. Methane conversion results obtained with this solid are presented as Example 8 in Table III below.

The results reported in the table are based on analyses of cumulative samples collected during the third, two-minute, methane contact run for each of these coprecipitated contact agents. Run conditions were 800°C and 860 GHSV.

TABLE III

| Example | wt.% Na in agent | % $CH_4$ conversion | % Selectivity | | | |
|---|---|---|---|---|---|---|
| | | | $C_{2-7}$ | CO | $CO_2$ | Coke |
| 6 | 9 | 2.0 | 92.6 | 2.5 | 3.0 | 1.9 |
| 7 | 2 | 7.7 | 93.1 | 4.5 | 2.1 | 0.3 |
| 8 | 2 | 15.2 | 78.1 | 4.0 | 17.9 | 0.0 |

Note that the agent of Example 8 had a higher Mn loading than either of the other agents. The effect was to substantially increase conversion. Further note that with respect to Examples 6 and 7, the lower Na loading of the agent employed in Example 7 is associated with generally superior results.

Examples 9—14 and Comparative Examples C and D

A number of oxidative synthesizing agents consisting of alpha-alumina-supported manganese oxides promoted with sodium were prepared by impregnating alumina support material with an appropriate amount of manganese (as manganese acetate) and an appropriate amount of sodium (as sodium acetate, unless otherwise noted) from water solutions. The impregnated solids were dried and calcined as described above in Examples 1—3. Results reported below in Table IV are based on analyses of 2-minute, cumulative samples collected during a methane contact run after the contact agent had equilibrated. Table IV also shows results obtained over otherwise equivalent, unpromoted oxidative synthesizing agents.

TABLE IV

Effect of sodium on manganese/alpha-alumina catalysts

| Example | Alumina support[a] | wt.% Mn | wt.% Na | Temperature (°C) Methane run | Regeneration | GHSV | % conversion | % Selectivity to $C_2$+ |
|---|---|---|---|---|---|---|---|---|
| C | A | 10 | — | 800 | 800 | 600 | 18 | 38 |
| D | A | 10 | — | 800 | 900 | 600 | 13 | 48 |
| 9 | A | 10 | 1.7 | 800 | 800 | 600 | 16.5 | 60 |
| 10 | A | 10 | 1.7 | 800 | 900 | 600 | 14 | 67 |
| 11 | B | 15 | 1.7[b] | 800 | 800 | 600 | 5.5 | 75 |
| 12 | B | 15 | 1.7 | 800 | 800 | 1200 | 15 | 30 |
| 13 | B | 15 | 6.7 | 800 | 800 | 600 | 35 | 30 |
| 14 | B | 15 | 6.7 | 800 | 800 | 1200 | 25 | 40 |

[a] Alumina support "A" is Norton 5551 alpha-alumina;
Alumina support "B" is UCI SAHT-99 alpha-alumina;

[b] Sodium was added during impregnation as a water solution of sodium pyrophosphate.

Examples 15—17 and Comparative Example E

The supported oxides of tin employed in the following examples were made by impregnation of the support with tin tartrate, provided as an aqueous solution of 7% hydrochloric acid. The solids were dried and then calcined in air at 700°C for 16 hours. To produce sodium-promoted oxidative synthesizing agents, these calcined solids were then impregnated with an appropriate amount of sodium, provided as aqueous solution of sodium acetate. The Na-impregnated solids were then dried and again calcined in air at 700°C for 16 hours. Results reported below in Table V are based on analyses of 2-minute cumulative samples collected during methane contact runs after the solids had equilibrated. Table V also shows results obtained over an otherwise equivalent, unpromoted oxidative synthesizing agent.

TABLE V
Effect of sodium on tin catalysts

| Example | Support | wt.% Sn | wt.% Na | Temp.°C | GHSV $hr.^{-1}$ | % conversion | % selectivity to $C_2$+ |
|---|---|---|---|---|---|---|---|
| E | $SiO_2$ | 10 | — | 800 | 600 | 4.8 | 37 |
| 15 | $SiO_2$ | 10 | 2 | 800 | 600 | 3.8 | 85 |
| 16 | $SiO_2$ | 10 | 2 | 900 | 600 | 8.5 | 73 |
| 17 | $Al_2O_3$ | 10 | 5 | 800 | 600 | 3.2 | 32 |

Example 18

A promoted oxidative synthesizing agent consisting of 5 wt.% $Na_4P_2O_7$/15 wt.% Mn on Houdry HSC 534 silica was prepared by impregnating the silica support with apprropiate amounts of manganese (as manganese acetate) and sodium pyrophosphate. Results obtained during a 2-minute methane contact run employing the equilibrated contact agent are described below in Table VI. The table shows both instantaneous results (based on analyses of samples collected at the times indicated) and cumulative results (based on analyses of accumulative sample collected over the entire run). Conditions for the methane contact were 800°C and 600 GHSV.

TABLE VI

| Run time (minutes) | % Conversion | % Selectivity $CH_2CH_2$ | $CH_3CH_3$ | $C_3$ | $C_4$—$C_7$ | CO | $CO_2$ | Coke |
|---|---|---|---|---|---|---|---|---|
| Instantaneous results | | | | | | | | |
| 1 | 22.1 | 48.5 | 25.1 | 6.6 | 4.5 | 0 | 15.2 | |
| 2 | 14.2 | 44.3 | 32.0 | 6.1 | 3.3 | 4.8 | 9.5 | |
| Cumulative results | | | | | | | | |
| 2 | 25.6 | 41.3 | 20.9 | 5.3 | 3.6 | 7.0 | 19.5 | 2.3 |

Example 19

This example demonstrates the effect of manganese oxide and sodium pyrophosphate loadings on methane conversion results. Four promoted oxidative synthesizing agents were examined: (1) 5%$Na_4P_2O_7$/10%Mn/$SiO_2$; (2) 5%$Na_4P_2O_7$/15%Mn/$SiO_2$; (3) 5%$Na_4P_2O_7$/20%Mn/$SiO_2$; and (4) 10%$Na_4P_2O_7$/20%Mn/$SiO_2$. Each of these agents were examined under a variety of methane contact conditions by varying contact temperature (generally from 650°—900°C) and space velocities (generally from 300—1200 GHSV). Figure 1 is a plot of $C_2$+ product selectivity vs. methane conversion for each of the solids. Results depicted in Figure 1 are based on analyses of 2-minute cumulative samples collected during the methane contact runs. As can be seen from the Figure, contact agent composition and operating conditions (temperature and space velocity) have significant effects on the results obtained. In general, higher temperatures and lower space velocities tend to enhance conversion for any given oxidative synthesizing agent whereas higher space velocities tend to enhance selectivities for any given oxidative synthesizing agent.

Example 20

This Example demonstrates the enhanced stability of oxidative synthesizing agents when P is incorporated into the composition. Two agents were compared: (1) 10%Mn/5%$Na_4P_2O_7$/$SiO_2$ and (2) 10%Mn/1.7%Na/$SiO_2$. The molar concentrations of Na in each of the two compositions were equivalent.

Both agents were prepared by impregnation using identical procedures except that $Na_4P_2O_7$ was the impregnating agent in the first solid and sodium acetate was the impregnating agent in the second solid. Both agents were placed in an automated reactor designed to operate according to the following cycle:

(1) Nitrogen purge—14 minutes;
(2) Methane feed—2 minutes;
(3) Nitrogen purge—14 minutes; and
(4) Air regeneration—30 minutes.

Each cycle thus required 1 hour for completion. Conditions for the methane contact portion of the cycle were 800°C and 420 GHSV for the Mn/Na/P agent and 800°C and 460 GHSV for the Mn/Na agent. Regenerations were also performed at 800°C in both cases. Figure 2 is a plot of methane conversion vs. Run Number during 1000 cycle tests conducted for each agent. Conversions shown in the Figure are based on analyses of 2-minute cumulative samples collected during the methane contact portion of the automated cycle.

As shown in the Figure, the presence of P in the oxidative synthesizing agent composition produces a less rapid decrease in activity with time.

Examples 21—23 and Comparative Example F.

Supported manganese oxides promoted by various alkaline earth metals were made by impregnating a silica support with the appropriate amount of manganese (as manganese acetate) and the appropriate amount of alkaline earth metal (also as the acetate) from water solutions. The support was Houdry HSC 534 silica. The impregnated solids were dried at 110°C for 4 hours and then calcined in air at 700°C for 16 hours. All calcined solids contained 10 wt.% Mn and each contained the same mole % of alkaline earth metal. Results reported below in Table VII are based on analyses of cumulative samples collected during the third, two-minute, methane-contact run for each promoted oxidative synthesizing agent. Run conditions were 800°C and 860 GHSV. Table VII also shows results obtained over an unpromoted oxidative synthesizing agent.

TABLE VII

| Example | Oxidative synthesizing agent | % $CH_4$ conversion | % $C_{2-7}$ selectivity |
|---|---|---|---|
| 1 | 10%Ba/10%Mn/$SiO_2$ | 13.4 | 65 |
| 2 | 3.0%Ca/10%Mn/$SiO_2$ | 3.1 | 76 |
| 3 | 1.8%Mg/10%Mn/$SiO_2$ | 8.6 | 67 |
| A | 10%Mn/$SiO_2$ | 7.6 | 56 |

Example 24

A contact solid comprising a reducible oxide of manganese was prepared by impregnating various magnesia supports with sodium permanganate, the amount of manganese provided being sufficient to yield a solid containing the equivalent of 10 wt.% $NaMnO_2/MgO$. The impregnated solids were dried at 110°C for four hours and then calcined in air at 800°C for 16 hours. A quartz tube reactor (12 mm. inside diameter) was charged with 10 ml. of the indicated solid. The reactor was brought up to reactor temperature (800°) under a flow of nitrogen. A feed of 100% methane was then contacted with the solids at about atmospheric pressure and a GHSV of 1200 $hrs^{-1}$. Results are reported below in Table VIII. Results reported are based on analyses of cumulative samples collected during methane contact runs of about 2 minutes.

TABLE VIII

| Magnesia support | % conversion | % Selectivity | | | | | |
|---|---|---|---|---|---|---|---|
| | | $C_2H_4$ | $C_2H_6$ | $C_{3-7}$ | CO | $CO_2$ | Coke |
| MagOx-95 (Basic chemical) | 21.61 | 14.72 | 25.4 | 2.51 | 0 | 57.25 | 0.12 |
| Martin marietta Mag Chem 500-G-3 | 12.76 | 2.05 | 7.90 | 0 | 0 | 15.88 | 14.17 |
| Michigan chemical MgO | 7.92 | 22.41 | 48.11 | 4.89 | 0 | 23.58 | 1.01 |
| Dart MgO | 15.8 | 33.3 | 33.4 | 21.9 | 0 | 11.3 | 0.1 |

While Table VIII indicates that results obtained over solids prepared from magnesias of various commercial suppliers may vary, it is presently believed that performance of relatively inferior magnesias may be improved by suitable activation procedures.

**Claims**

1. A method for converting methane to higher hydrocarbon products which comprises contacting a gas comprising methane at synthesising conditions with at least one reducible oxide of at least one metal, which oxide when contacted with methane at synthesizing conditions is reduced and produces higher hydrocarbon products and water, characterised in that the contacting is conducted in the presence of a promoting amount of at least one promoter (a) selected from alkali metals and compounds thereof optionally together with at least one promoter (b) selected from alkaline earth metals and compounds thereof, said reducible oxide and said promoter or promoters optionally being associated with a support material.

2. A method as claimed in claim 1 characterised in that said support is selected from alkaline earth metals and compounds thereof.

3. A method as claimed in claim 2 characterised in that said support comprises magnesia.

4. A method as claimed in any one of claims 1 to 3 characterised in that said at least one promoter (a) is selected from Li, Na, K, Rb, Cs, and compounds thereof.

5. A method as claimed in any one of claims 1 to 4 characterised in that said at least one promoter (b) is selected from Mg, Ca, Sr, Ba and compounds thereof.

6. A method for converting methane to higher hydrocarbon products which comprises contacting a gas comprising methane at synthesising conditions with at least one reducible oxide of at least one metal, which oxide when contacted with methane at synthesizing conditions is reduced and produces higher hydrocarbon products and water, characterised in that the contacting is conducted in the presence of a promoting amount of at least one promoter selected from alkaline earth metals and compounds thereof, said contacting being effected in the absence of catalytically effective Ni, Rh, Pd, Ag, Os, Ir, Pt, Au and compounds thereof, and said reducible oxide and said promoter optionally being associated with a support material.

7. A method as claimed in claim 6 characterised in that said at least one promoter is selected from Mg, Ca, Sr, Ba and compounds thereof.

8. A method as claimed in any one of claims 1 to 7 characterised in that said support material comprises silica.

9. A method as claimed in any one of claims 1 to 8 characterised in that said at least one reducible oxide of at least one metal is selected from reducible oxides of Mn, Sn, In, Ge, Pb, Sb and Bi.

10. A method as claimed in any one of claims 1 to 8 characterised in that said at least one reducible oxide of at least one metal is an oxide of manganese.

11. A method as claimed in any one of claims 1 to 10 characterised in that said contacting is effected in the presence of a stabilising amount of phosphorus.

12. A method as claimed in any one of claims 1 to 11 characterised in that it comprises:
(i) effecting the contacting of the gas comprising methane in a first zone to form higher hydrocarbons, co-product water, and solids comprising reduced metal oxide;
(ii) recovering higher hydrocarbons,
(iii) at least periodically contacting said solids comprising reduced metal oxide with an oxygen-containing gas in a second zone to produce a solid comprising a reducible metal oxide; and
(iv) returning said solid comprising a reducible metal oxide from the second zone to the first zone.

**Patentansprüche**

1. Verfahren zur Umwandlung von Methan in höhere Kohlenwasserstoffprodukte, bei dem man ein Gas, das Methan enthält, bei Synthesebedingungen mit mindestens einem Oxid von mindestens einem Metall in Kontakt bringt, wobei das Oxid, wenn es mit Methan bei Synthesebedingungen in Kontakt gebracht wird, reduziert wird, und höhere Kohlenwasserstoffprodukte und Wasser ergibt, dadurch gekennzeichnet, daß das Inkontaktbringen in Gegenwart einer beschleunigenden Menge mindestens eines Beschleunigers erfolgt, ausgewählt aus (a) Alkalimetallen und Verbindungen davon, gegebenenfalls zusammen mit mindestens einem Beschleuniger, ausgewählt aus (b) Erdalkalimetall und Verbindungen davon, wobei das reduzierbare Oxid und der Beschleuniger oder die Beschleuniger gegebenenfalls mit einem Trägermaterial verbunden sind.

2. Verfahren nach Anspruch 1, dadurch gekennzeichnet, daß das Trägermaterial aus Erdalkalimetallen und deren Verbindungen ausgewählt wird.

3. Verfahren nach Anspruch 2, dadurch gekennzeichnet, daß das Trägermaterial Magnesium enthält.

4. Verfahren nach einem der Ansprüche 1 bis 3, dadurch gekennzeichnet, daß mindestens ein Beschleuniger (a) aus Li, Na, K, Rb, Cs und deren Verbindungen ausgewählt wird.

5. Verfahren nach einem der Ansprüche 1 bis 4, dadurch gekennzeichnet, daß mindestens ein Beschleuniger (b) aus Mg, Ca, Sr, Ba und deren Verbindungen ausgewählt wird.

6. Verfahren zur Umwandlung von Methan in höhere Kohlenwasserstoffprodukte, bei dem man ein Gas, das Methan enthält, bei Synthesebedingungen mit mindestens einem reduzierbaren Oxid von mindestens einem Metall in Kontakt bringt, wobei das Oxid, wenn es mit Methan bei Synthesebedingungen in Kontakt gebracht wird, reduziert wird und höhere Kohlenwasserstoffprodukte und Wasser ergibt, dadurch gekennzeichnet, daß das Inkontaktbringen in Gegenwart einer beschleunigenden Menge mindestens eines Beschleunigers, ausgewählt aus Erdalkalimetallen und Verbindungen davon durchgeführt wird, wobei das Inkontaktbringen in Abwesenheit von katalytisch wirksamen Ni, Rh, Pd, Ag, Os, Ir, Pt, Au und deren Verbindungen durchgeführt wird und wobei das reduzierbare Oxid und der Beschleuniger gegebenenfalls mit einem Trägermaterial verbunden sind.

7. Verfahren nach Anspruch 6, dadurch gekennzeichnet, daß mindestens ein Beschleuniger aus Mg, Ca, Sr, Ba und deren Verbindungen ausgewählt wird.

8. Verfahren nach einem der Ansprüche 1 bis 7, dadurch gekennzeichnet, daß das Trägermaterial Siliziumdioxid enthält.

9. Verfahren nach einem der Ansprüche 1 bis 8, dadurch gekennzeichnet, daß mindestens ein reduzierbares Oxid mindestens eines Metalls aus den reduzierbaren Oxiden von Mn, Sn, In, Ge, Pb, Sb und Bi ausgewählt wird.

10. Verfahren nach einem der Ansprüche 1 bis 8, dadurch gekennzeichnet, daß mindestens ein reduzierbares Oxid von mindestens einem Metall ein Oxid von Mangan ist.

11. Verfahren nach einem der Ansprüche 1 bis 10, dadurch gekennzeichnet, daß das Inkontaktbringen in Gegenwart einer stabilisierenden Menge von Phosphor durchgeführt wird.

12. Verfahren nach einem der Ansprüche 1 bis 11, dadurch gekennzeichnet, daß man:

(i) das Inkontaktbringen des Gases, das Methan enthält, in einer ersten Zone durchführt um höhere Kohlenwasserstoffe, das Nebenprodukt Wasser und Feststoffe, enthaltend reduzierte Metalloxide, zu bilden;

(ii) die höheren Kohlenwasserstoffe zurückgewinnt;

(iii) die Feststoffe, die reduziertes Metalloxid enthalten, mindestens periodisch mit einem sauerstoffhaltigen Gas in einer zweiten Zone in Kontakt bringt, um einen Feststoff, der ein reduzierbares Metalloxid enthält, zu bilden; und

(iv) den Feststoff, der ein reduzierbares Metalloxid enthält, aus der zweiten Zone in die erste Zone zurückführt.

**Revendications**

1. Procédé pour convertir du méthane en produits hydrocarbonés supérieurs qui comprend la mise en contact d'un gaz comprenant du méthane dans des conditions de synthèse avec au moins un oxyde réductible d'au moins un métal, lequel oxyde est réduit lorsqu'il est mis en contact avec du méthane dans des conditions de synthèse et produit des produits hydrocarbonés supérieurs et de l'eau, caractérisé en ce que le contact est effectué en présence d'une quantité d'amorçage d'au moins un promoteur (a) choisi parmi des métaux alcalins et leurs composés, éventuellement avec au moins un promoteur (b) choisi parmi des métaux alcalino-terreux et leurs composés, cet oxyde réductible et ce promoteur ou ces promoteurs étant éventuellement associés à un matériau support.

2. Procédé suivant la revendication 1, caractérisé en ce que ce support est choisi parmi des métaux alcalino-terreux et leurs composés.

3. Procédé suivant la revendication 2, caractérisé en ce que ce support comprend de la magnésie.

4. Procédé suivant l'une quelconque des revendications 1 à 3, caractérisé en ce que ce promoteur (a) qui est au moins présent, est choisi parmi Li, Na, K, Rb, Cs et leurs composés.

5. Procédé suivant l'une quelconque des revendications 1 à 4, caractérisé en ce que ce promoteur (b) qui est au moins présent, est choisi parmi Mg, Ca, Sr, Ba et leurs composés.

6. Procédé pour convertir du méthane en produits hydrocarbonés supérieurs qui comprend la mise en contact d'un gaz comprenant du méthane dans des conditions de synthèse avec au moins un oxyde réductible d'au moins un métal, lequel oxyde est réduit lorsqu'il est mis en contact avec du méthane dans des conditions de synthèse et produit des produits hydrocarbonés supérieurs et de l'eau, caractérisé en ce que le contact est effectué en présence d'une quantité d'amorçage d'au moins un promoteur choisi parmi des métaux alcalino-terreux et leurs composés, ce contact étant effectué en l'absence de Ni, Rh, Pd, Rg, Os, Ir, Pt, Ru et de leurs composés efficaces du point de vue catalytique, et cet oxyde réductible et ce promoteur étant éventuellement associés à un matériau support.

7. Procédé suivant la revendication 6, caractérisé en ce que ce promoteur qui est au moins présent, est choisi parmi Mg, Ca, Sr, Ba et leurs composés.

8. Procédé suivant l'une quelconque des revendications 1 à 7, caractérisé en ce que ce matériau support comprend de la silice.

9. Procédé suivant l'une quelconque des revendications 1 à 8, caractétrisé en ce que cet oxyde métallique au moins présent d'au moins un métal, est choisi parmi les oxydes réductibles de Mn, Sn, In, Ge, Pb, Sb et Bi.

10. Procédé suivant l'une quelconque des revendications 1 à 8, caractérisé en ce que cet oxyde métallique au moins présent d'au moins un métal, est un oxyde de manganèse.

11. Procédé suivant l'une quelconque des revendications 1 à 10, caractérisé en ce que le contact est effectué en présence d'une quantité stabilisante de phosphore.

12. Procédé suivant l'une quelconque des revendications 1 à 11, caractérisé en ce que ce qu'il comprend:

(i) la mise en contact du gaz comprenant du méthane dans une première zone, pour former des hydrocarbures supérieurs, de l'eau co-produite et des solides comprenant un oxyde métallique réduit,

(ii) la récupération des hydrocarbures supérieurs,

(iii) le contact au moins périodique de ces solides comprenant l'oxyde métallique réduit avec un gaz contenant de l'oxygène dans une seconde zone, pour produire un solide comprenant un oxyde métallique réductible; et

(iv) le renvoi de ce solide comprenant un oxyde métallique réductible de la seconde zone à la première zone.

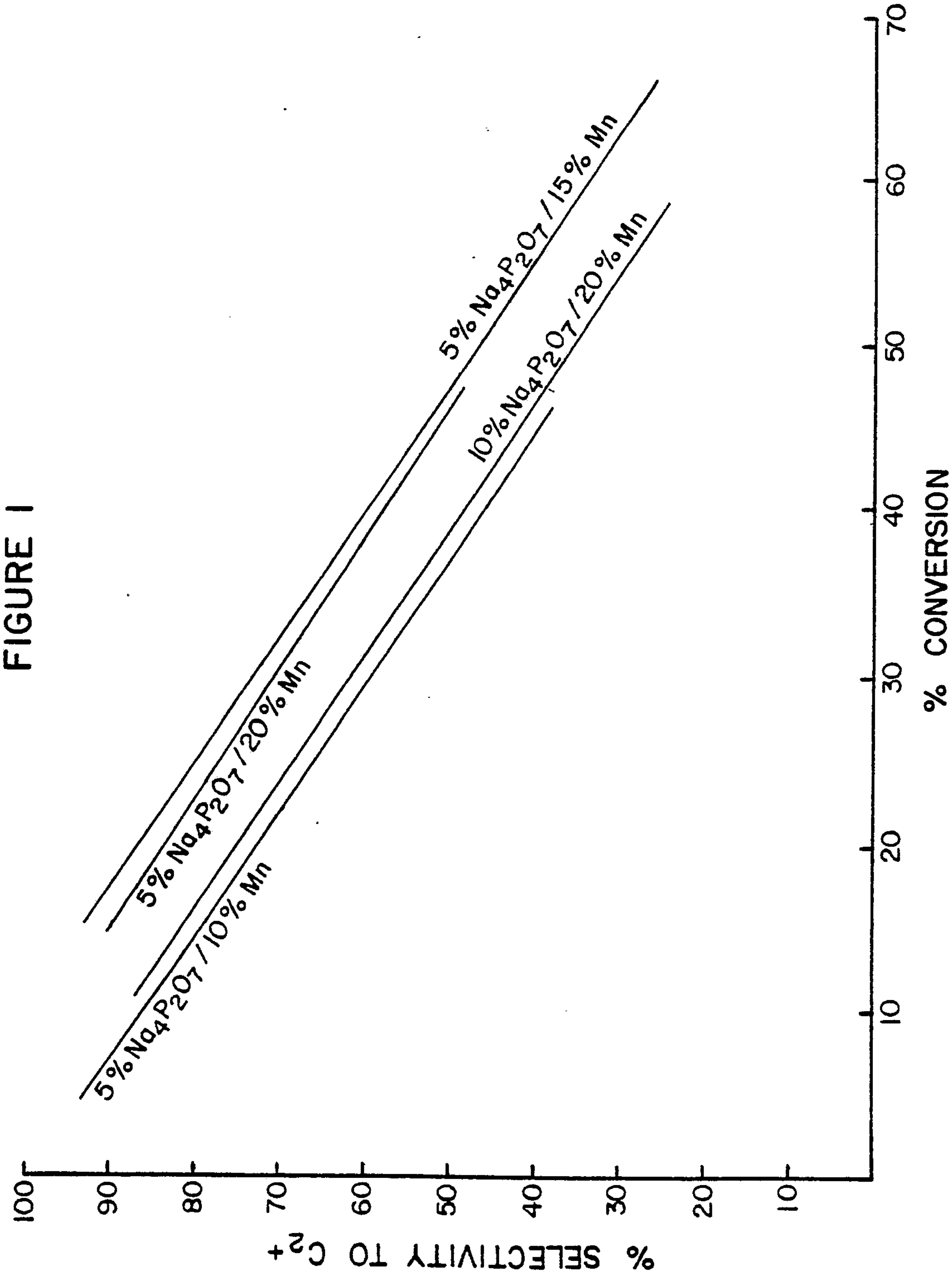
5% $Na_4P_2O_7$ / 15% Mn
10% $Na_4P_2O_7$ / 20% Mn
5% $Na_4P_2O_7$ / 20% Mn
5% $Na_4P_2O_7$ / 10% Mn
% SELECTIVITY TO $C_2$+
100
90
80
70
60
50
40
30
20
10
% CONVERSION
10
20
30
40
50
60
70

FIGURE I

FIGURE 2

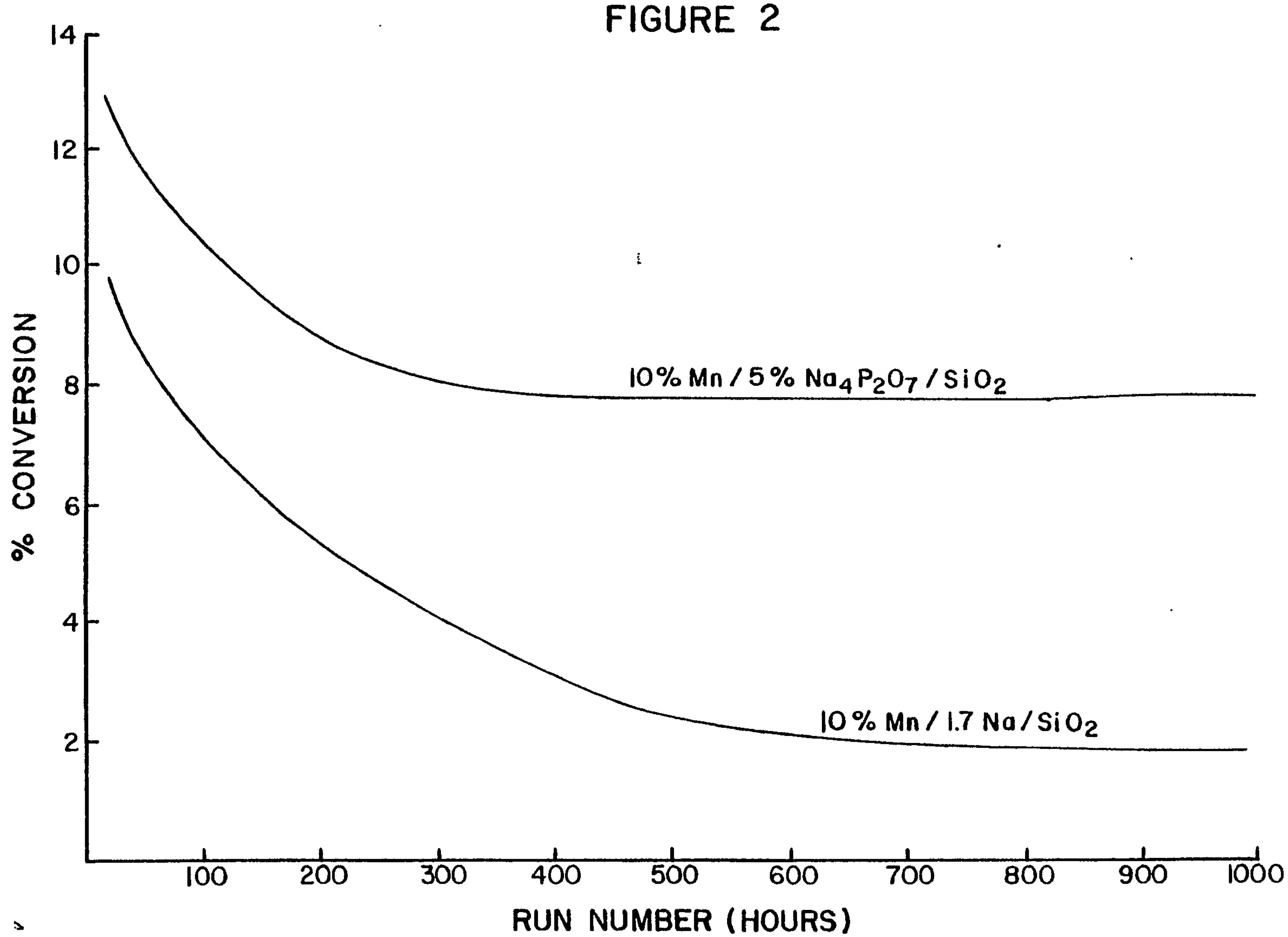
% CONVERSION
14
12
10
8
6
4
2
100
200
300
400
500
600
700
800
900
1000
RUN NUMBER (HOURS)
10% Mn / 5% $Na_4P_2O_7$ / $SiO_2$
10% Mn / 1.7 Na / $SiO_2$